# EUROPÄISCHE PATENTANMELDUNG

(11) **EP 1 126 031 A1**
(43) Veröffentlichungstag der Anmeldung: **22.08.2001**
(21) Anmeldenummer: 01103893.2
(22) Anmeldetag: 16.02.2001
(51) Int. Cl.: C12N 15/86

(54) **Nichthumaner helferabhängiger Virusvektor**

(30) Priorität: 16.02.2000 DE 10006886
(71) Anmelder: DeveloGen Aktiengesellschaft für entwicklungsbiologische Forschung, 37079 Göttingen (DE)
(72) Erfinder: Hillgenberg, Moritz, Dr., 13347 Berlin-Mitte (DE); Hofmann, Christian, Dr., 10439 Berlin (DE); Löser, Peter, Dr., 10439 Berlin (DE)
(74) Vertreter: Weiss, Wolfgang, Dipl.-Chem. Dr.

(57) **Zusammenfassung**

Die Erfindung betrifft einen nichthumanen helferabhängigen Virusvektor für den Transfer von Nukleinsäuresequenzen. Anwendungsgebiete sind die Medizin, die Veterinärmedizin, die Biotechnologie und die Gentechnik.

## Beschreibung

Die Erfindung betrifft einen nichthumanen helferabhängigen Virusvektor für den Transfer von Nukleinsäuresequenzen. Anwendungsgebiete sind die Medizin, die Veterinärmedizin, die Biotechnologie und die Gentechnik.

In den vergangenen Jahren sind zahlreiche Methoden und Vektoren für den Gentransfer mit dem Ziel einer Gentherapie oder Vakzinierung entwickelt worden (Übersicht bei: Verma, M.I. und Somia, N. (1997). Nature 389, 239-242). Dabei werden vor allem Vektoren, die von Retroviren, Adeno-assoziierten Viren (AAV) oder humanen Adenoviren abgeleitet sind, für einen Gentransfer mit dem Ziel einer Gentherapie favorisiert. Diese Vektortypen haben ein breites Spektrum effizient infizierbarer Zelltypen und sind daher für den Gentransfer in verschiedene Gewebe geignet.

Die sog. adenoviralen Vektoren der ersten Generation wurden über das letzte Jahrzehnt intensiv als Gentransfer-Vektoren erforscht (Übersicht bei: Bramson, J.L. et al. (1995). Curr. Op. Biotech. 6, 590-595). Sie leiteten sich vom humanen Adenovirus des Serotyps 5 ab und sind in der essentiellen E1-Region, oft auch in der nicht-essentiellen E3-Region deletiert, wodurch bis zu 8 KBp Fremd-DNA in das Virusgenom inseriert werden können. Diese Vektoren können auf die E1-Defizienz komplementierenden Zellen zu hohen Titern produziert werden, lassen sich gut lagern und vermitteln in vitro und in vivo einen effektiven Gentransfer. In vivo kommt es aber zu einem schnellen Verlust der Expression der Transgene. Ferner wurde teilweise massive Gewebe-Toxizität nach Applikation hoher Vektor-Dosen beobachtet. Beides wird zumindest zum Teil durch immunologische Reaktionen auf die im Vektor verbliebenen viralen Gene verursacht. Es wurde daher versucht, vor allem weitere frühe virale Gene auszulagern, eine entscheidende Verbesserung des in vivo-Gentransfers konnte damit aber nicht erreicht werden.

Neuerdings entwickelte, vollständig rekombinante humane Adenovirus-Vektoren (Kochanek, S. et al. (1996). Proc. Natl. Acad. Sci. USA 93; 5731 - 5736) zeigten in Tiermodellen einen effektiven Gentransfer bei reduzierter Toxizität und eine verlängerte Transgenexpression (Morral, N. et al. (1998), Hum. Gen. Ther. 9: 2709-2716). Da aber auch diese Vektoren die normale Hülle des humanen Adenovirus sowie im Vektorgenom die für Replikation und Verpackung notwendigen cis-Elemente - die Inverted Terminal Repeats (ITRs) und das Verpackungssignal - aufweisen, bleiben auch bei diesen Vektoren zwei Limitierungen bestehen, die als die Hauptprobleme humaner Adenovirus-Vektoren gelten können. Beide liegen im menschlichen Ursprung der verwendeten Adenoviren und der weiten Verbreitung derselben in der menschlichen Bevölkerung begründet: (1) Die zumeist vorhandenen präexistierenden Antikörper führen zu weitgehender Neutralisierung des Vektors. Aktuelle Studien in Tiermodellen haben gezeigt, daß ein effizienter Gentransfer bei präexistierenden Antikörpern nur bei Einsatz hoher Vektor-Dosen möglich ist (Nunes, F.A. et al. (1999), Hum. Gen. Ther. 10, 2515-2526), was jedoch zu starken toxischen Nebenwirkungen, unter anderem auch gravierenden hämatologischen Nebeneffekten führen kann (Cichon, G. et al. (1999), J. Gene Med. 1, 360-371). (2) Zudem besteht die Gefahr einer Koreplikation des rekombinanten Vektors bei einer natürlichen Infektion mit einem Wildtyp-Adenovirus mit nicht einschätzbaren Folgen. Derzeitige humane Adenovirus-Vektoren können daher für eine Anwendung zur Gentherapie beim Menschen nur eingeschränkt in Betracht gezogen werden. Ein Ansatz zur Überwindung des Problems der präexistierenden Antikörper gegen humane Adenoviren wurde in neuerer Zeit durch die Entwicklung adenoviraler Vektoren nichthumanen Ursprungs gefunden. Das virale Genom ist bei diesen Vektoren weitgehend unverändert, in der Regel wurden kleinere, für die Virus-Propagierung nicht-essentielle Regionen durch ein Transgen ersetzt (Mittal, S.K. et al. (1995), J.Gen. Virol. 76, 93-102; Klonjkowski, B. et al. (1997), Hum. Gen. Ther. 8: 2103-2115; Michou, I. et al. (1999). J. Virol. 72, 1399-1410. Diese Vektoren haben aber eine auf nur wenige KBp begrenzte Insertionskapazität für das Transgen, sind bezüglich ihrer Biologie zumeist wenig erforscht und sind vor allem hinsichtlich ihrer Sicherheit im menschlichen Organismus bislang nicht charakterisiert. Verpackungssignale nichthumaner Adeonivren wurden bislang nicht lokalisiert.

Eine Aufgabe der vorliegenden Erfindung was somit die Bereitstellung neuartiger Vektoren für den Gentransfer, bei denen die Nachteile bisheriger Vektoren, insbesondere Vorhandensein einer präexistenten Immunantwort im Menschen, limitierte Fremd-DNA Kapazität oder/und mögliche Nebeneffekte aufgrund von bisher nicht oder nur wenig charakterisierten Virusgenen zumindest teilweise beseitigt sind.

Ein erster Aspekt der Erfindung betrifft einen Vektor insbesondere zur Klonierung von viralen Genomen, z.B. von Genomen nichthumaner Adenoviren sowie dessen Verwendung zur Identifizierung und Charakterisierung von Verpackungssequenzen der nichthumanen Adenoviren, sowie zur Herstellung von adenoviralen Gentransvektoren. Dieser Klonierungsvektor umfaßt
(a) eine Verpackungssequenz eines Bakteriophagen,
(b) mindestens eine Klonierungsstelle zur Insertion heterologer Nukleinsäuresequenzen,
(c) mindestens zwei Schnittstellen für eine Restriktionsendonuklease, die beidseitig die Klonierungsstelle (b) umrahmen,
(d) einen bakteriellen Replikationsursprung, und
(e) ein bakterielles Selektionsmarkergen.

Der Klonierungsvektor enthält Elemente, die eine Propagierung und Selektion in prokaryontischen Wirtsorganismen, insbesondere in gram-negativen Bakterien wie etwa E.coli erlauben, insbesondere einen bakteriellen Replikationsursprung, beispielsweise der ColE1 Replikationsursprung aus E.coli sowie ein bakterielles Selektionsmarkergen, insbesondere ein Antibiotikaresistenzgen, wie etwa das Ampicillin-Resistenzgen. Der Vektor enthält eine Klonierungsstelle zur Insertion heterologer Nukleinsäuresequenzen, insbesondere heterologer Nukleinsäuresequenzen mit einer Länge von ≥ 10 kBp. Der Vektor ist vorzugsweise ein Bakteriophagen- oder Cosmidvektor. Er enthält vorzugsweise die Bakteriophagen-Verpackungssequenzen von einem lambdoiden Phagen, insbesondere das cos-Element des Phagen Lambda.

Die beidseitig die Klonierungsstelle umrahmenden Restriktionsschnittstellen sind vorzugsweise die Schnittstellen für eine Restriktionsendonuklease mit einer Erkennungsspezifitätvon mindestens 8 Basenpaaren, insbesondere die Schnittstellen für eine Meganuklease wie etwa I-Scel.

Die Klonierungsstelle ist vorzugsweise eine multiple Klonierungsstelle, d.h. sie enthält mehrere vorzugsweise singuläre Schnittstellen für Restriktionsendonukleasen.

In die Klonierungsstelle des Vektors kann das Genom eines nichthumanen Adenovirus inseriert werden, d.h. eines Adenovirus, das natürlicherweise in einer nichthumanen Spezies vorkommt, die beispielsweise ausgewählt ist aus Säugern und Vögeln, wie aufgeführt in Russell, W.C. und Benkö, M. (1999), Adenoviruses (Adenoviridae): Animal viruses. In: Granoff, A. und Webster, R.G. (Eds): Encyclopedia of Virology. Academic Press, London, und zwar vor allem Adenoviren aus Affen (SAV, verschiedene Serotypen), Ziegen (caprine, verschiedene Serotypen), Hunden (CAV, verschiedene Serotypen), Schweinen (PAV, verschiedene Serotypen), Rindern (BAV, verschiedene Serotypen, Schafen (OAV1-6 und OAV287) und Hühnern (FAV, verschiedene Serotypen) und EDS-Virus. Vorzugsweise ist das Virus ein Adenovirus vom Schaf oder Rind. Beispiele für geeignete Schafadenoviren sind ovine Mastadenoviren oder ovine Atadenoviren wie etwa das OAV-lsolat 287, dessen Nukleotidsequenz unter der Genbank Acc. No. U40389 angegeben ist. Geeignete Rinder-Adenoviren sind bovine Atadenoviren oder bovine Mastadenoviren. Dabei sind im Komplement-Fixierungsassay negative bovine und ovine Atadenoviren besonders interessant.

Der erfindungsgemäße Klonierungsvektor kann zur Herstellung von partiell deletierten Genomen nichthumaner Adenoviren verwendet werden. Hierzu kann das in den Vektor inserierte Virusgenom durch Spaltung mit geeigneten Restriktionsendonukleasen oder Kombinationen von Restriktionsendonukleasen partiell deletiert werden. Auf diese Weise kann die Lage der viralen Verpackungssequenzen im Genom charakterisiert werden.

Insbesondere wird in den erfindungsgemäßen Phagen- oder Cosmidvektor das virale Genom in die Klonierungsstelle, d.h. zwischen den beiden Schnittstellen für eine Meganuklease inseriert und die Infektiösität der daraus durch Meganukleaseverdau freigesetzten Virusgenome nach Transfektion in eine Verpackungszellinie bestimmt. Auf Basis des das Virusgenom enthaltenden Phagen- und Cosmidvektors werden Deletionsmutanten hergestellt, in denen verschiedene Teile des viralen Genoms durch- heterologe Nukleinsäuren, beispielsweise durch nichtkodierende DNA sowie eine Reportergenkassette ersetzt sind. Die Deletionsmutanten werden nach Meganukleaseverdau in eine Verpackungszellinie transfiziert, die anschließend mit einem Helfervirus infiziert und nach Auftreten des cytopathischen Effekts lysiert wird. Mit den resultierenden Lysaten werden Zellen infiziert. Zellen, die das Reportergen exprimieren, enthalten ein corepliziertes und verpacktes helferabhängiges nichthumanes Virus. Auf diese Weise kann derjenige Bereich des Virusgenoms eingegrenzt werden, der neben den ITRs für die Herstellung von das Reportergen transduzierenden infektiösen Partikeln im viralen Genom verbleiben muß (Verpackungssequenz). Durch weitere Deletionen kann die Lage der Verpackungssequenz nach der gleichen Methode genauer charakterisiert werden.

Ein weiterer Aspekt der Erfindung ist somit ein Verfahren zur Charakterisierung von Verpackungssequenzen nichthumaner Adenovirus-vektoren, wobei
(a) ein vorbestimmter Bereich des in den Klonierungsvektor inserierten viralen Genoms deletiert und durch eine heterologe Nukleinsäure umfassend eine Reportergenkassette ersetzt wird,
(b) die gemäß (a) erzeugten Konstrukte in ein Helfersystem eingebracht werden, das zur Replikation und Verpackung des nichthumanen Adenovirus notwendigen Genprodukte bereitstellt,
(c) bestimmt wird, ob mit dem System gemäß (b) nichthumane Adenoviruspartikel entstehen, die das Reportergen enthalten, und
(d) gegebenenfalls die Schritte (a), (b) und (d) wiederholt werden, bis die neben den Inverted Terminal Repeat (ITRs) für eine Verpackung notwendigen cis-Sequenzen des Virusgenoms (Verpackungssequenzen) charakterisiert sind.

Die heterologe Nukleinsäure, die den deletierten Teil des viralen Genoms ersetzt, hat vorzugsweise eine ähnliche Länge wie der deletierte Abschnitt und umfaßt vorzugsweise eine genomische DNA, beispielweise eine nichtkodierende genomische Säuger DNA, insbesondere eine nichtkodierende genomische humane DNA. Weiterhin enthält die heterologe Nukleinsäure eine Reportergenkassette, d.h. ein für ein nachweisbares Polypeptid kodierendes Gen in exprimierbarer Form, beispielsweise das E.coli lac-Z Gen oder ein für ein Fluoreszenzprotein, beispielsweise GFP, kodierendes Gen.

Das auf diese Weise erzeugte Konstrukt wird ein Helfersystem eingebracht, das zur Replikation und Verpackung des nichthumanen Adenovirus notwendige Genprodukte bereitstellt. Dieses Helfersystem umfaßt eine für das jeweilige Virus permissive Zellinie, beispielsweise die Zellinie CSL503 (Pye et al., Austr. Vet. J. 66 (1989), 231-232) für das Schaf-Adenovirus OAV 287. Weiterhin umfaßt das Helfersystem ein Helfervirus, beispielsweise das OAV 287 Wildtypvirus, welches die für die Verpackung des deletierten Konstrukts notwendigen Genprodukte exprimiert. Aus dem Helfersystem, z.B. nach Zellyse gewonnene virale Partikel werden anschließend in einem neuen Infektionszyklus daraufhin untersucht, ob sie das Reportergen exprimieren. Auf diese Weise kann derjenige Bereich des Virusgenoms eingegrenzt werden, der neben den ITR-Bereichen für die Herstellung von das Reportergen transduzierenden infektiösen Partikeln im viralen Genom verbleiben muß, d.h. die Verpackungssequenz. Durch die aus einem solchen Experiment gewonnenen Erkenntnisse lassen sich durch Herstellung von weiteren viralen Deletionsvarianten die Lage der Verpackungssequenz genau charakterisieren. Auf diese Weise konnte beispielsweise die Lage der Verpackungssequenz des Schaf-Adenovirusisolats OAV287 auf die 5-terminalen 2,8 kBp oder die 3'-terminalen 1 kBp des Virusgenoms eingegrenzt werden.

Durch Charakterisierung der Verpackungssequenz können neuartige nichthumane helferabhängige Adenovirusvektoren mit partieller oder vollständiger Deletion der kodierenden Virusgene hergestellt werden, wobei die für Replikation und Verpackung des Vektors notwendigen cis-Elemente, d.h. die ITR-Bereiche und die Verpackungssequenz erhalten bleiben. Dieser neuartige Vektortyp hat gegenüber bislang existierenden viralen Vektorsystemen entscheidende Vorteile. Es lassen sich rekombinante Deletionsvarianten aus dem gesamten Spektrum nichthumaner Adenoviren herstellen, so daß eine Vielfalt von geeigneten helferabhängigen Adenovirusvektoren erzeugt werden kann, die den Ansprüchen an einen Vektor für den Gentransfer, insbesondere für eine Vakzinierung und die Gentherapie, genügt und für entsprechende Anwendungen auch im Menschen einsetzbar ist.

Aus dem in den erfindungsgemäßen Phagen- oder Cosmidvektor inserierten Genom des helferabhängigen nichthumanen Virus, in dem alle notwendigen cis-Elemente für die Replikation (ITRs) und Verpackung (Verpackungssequenz) in Form geeigneter viraler 5'- und 3'-Enden enthalten sind, während andere Regionen des Virus teilweise oder vollständig deletiert werden, kann ein Basisvektor hergestellt werden, der wiederum zur Herstellung eines viralen Vektors für den Gentransfer geeignet ist. In den Basisvektor wird eine Klonierungsstelle zur Insertion von heterologer DNA mit oder ohne Größenausgleich, beispielsweise eine multiple Klonierungsstelle oder ein größeres Stück heterologer nichtfunktioneller, vorzugsweise nichtkodierender DNA mit geeigneten Restriktionsschnittstellen zwischen die viralen 5'- und 3'-Bereiche des viralen Genoms eingefügt. Gegebenenfalls kann eine Reportergenexpressionskassette in eine der Schnittstellen in der Insertionsstelle eingefügt werden, um eine erleichterte Detektion und Titerbestimmung der zu generierenden helfer-abhängigen nichthumanen Viren zu ermöglichen. Der Basisvektor ist vorzugsweise ein Phagen- oder Cosmidvektor, aus dem durch Restriktionsspaltung vorzugsweise durch Spaltung mit einer Meganuklease das virale Genom des Gentransfervektors freigesetzt werden kann.

Ein weiterer Aspekt der Erfindung ist somit ein Basisvektor insbesondere zur Herstellung eines viralen Vektors für den Gentransfer, umfassend
(a) eine Verpackungssequenz eines Bakteriophagen,
(b) virale Sequenzen umfassend zwei Inverted Terminal Repeats (ITRs) und eine oder mehrere Verpackungssequenzen eines nichthumanen Adenovirus, wobei die viralen Sequenzen nicht in der Lage sind, eine helferunabhängige virale Replikation und Verpackung in einer permissiven Zellinie zu bewirken,
(c) eine Klonierungsstelle mit oder ohne Größenausgleich zur Insertion von heterologer DNA und gegebenenfalls eine Reportergenkassette, die innerhalb der viralen Sequenzen (b) liegen,
(d) mindestens zwei Schnittstellen für eine Restriktionsendonuklease, die beidseitig die viralen Sequenzen (b) umrahmen,
(e) einen bakteriellen Replikationsursprung und
(f) ein bakterielles Selektionsmarkergen,
wobei die Sequenzen (a), (e) und (f) außerhalb des von den Schnittstellen (d) umrahmten Sequenzbereichs liegen.

Die Klonierungsstelle kann eine Insertionsstelle mit Größenausgleich sein, die aus einer nichtkodierenden DNA-Sequenz mit einer Länge von 10 bis 30 kBp besteht. Diese nichtkodierende DNA-Sequenz kann eine genomische Sequenz, beispielsweise eine chromosomale DNA-Sequenz aus dem menschlichen Genom mit mindestens zwei dualen Schnittstellen sein. Bei Insertion einer heterologen Nukleinsäuresequenz in die Klonierungsstelle können unterschiedlich große Teile der zum Größenausgleich vorhandener DNA-Sequenz deletiert werden.

Aus diesem Basisvektor kann ein helferabhängiger nichthumaner Adenovirusvektor mit einem Transgen hergestellt werden, beispielsweise indem eine Expressionskassette für das Transgen in die Klonierungsstelle des Basisvektors inseriert und dann das virale Genom aus dem Basisvektor herausgeschnitten wird.

In den Basisvektor kann eine geeignete Transgen-Expresssionskassette in die Klonierungsstelle beispielsweise durch Cosmidklonierung eingefügt werden. Das erhaltene Konstrukt kann nach Meganukleasespaltung im Helfersystem transfiziert und das durch das Helfersystem replizierte und verpackte helferabhängige nichthumane Virus durch Lyse der Zellen gewonnen werden. Durch wiederholtes Einbringen des das helferabhängige nichthumane Virus enthaltenden Zellysats in das Helfersystem kann das helferabhängige nichthumane Virus amplifiziert werden.

Ein weiterer Aspekt der Erfindung ist somit ein viraler Gentransvektor umfassend die Hülle eines nichthumanen Adenovirus und darin verpacktes genetisches Material, das
(a) virale Sequenzen umfassend zwei Inverted Terminal Repeats (ITRs) und eine oder mehrere Verpackungssequenzen eines nichthumanen Adenovirus,
(b) eine oder mehrere Nukleinsäuresequenzen, die für bezüglich des nichthumanen Adenovirus heterologe Peptide oder Polypeptide kodieren, operativer Verknüpfung mit Expressionskontrollsequenzen und
(c) gegebenenfalls eine nichtkodierende DNA
enthält,
wobei das genetische Material nicht in der Lage ist, eine helferunabhängige virale Replikation und Verpackung in einer permissiven Zellinie zu bewirken.

Die viralen Sequenzen des Basis- und des Gentransfervektors enthalten vorzugsweise nur die für Replikation und Verpackung in die Hülle notwendigen cis-Elemente des Virusgenoms und sind ansonsten im wesentlichen frei von funktionellen Nukleinsäuresequenzen des nichthumanen Adenovirus, insbesondere von Nukleinsäuresequenzen, die für zur Replikation und Verpackung des Virus notwendige Genprodukte kodieren. Besonders bevorzugt enthalten die viralen Sequenzen ≤ 10 kb und insbesondere ≤5 kb an Nukleinsäuresequenzen, die aus dem Wildtypvirus stammen.

Das genetische Material des Gentransfervektors enthält Nukleinsäuresequenzen, die für bezüglich des nichthumanen Adenovirus heterologe Peptide oder Polypeptide kodieren, in operativer Verknüpfung mit Expressionskontrollsequenzen, insbesondere mit Expressionskontrollsequenzen, die eine Expression in Säugerzellen beispielsweise in humanen Zellen erlauben. Die Expressionskontrollsequenzen können entweder konstitutiv in der gewünschten Zielzelle aktiv oder regulierbar sein. Die Expressionskontrollsequenzen können viralen oder zellulären Ursprungs sein oder eine Kombination viraler und zellulärer Elemente umfassen. Beispiele für geeignete Promotoren sind virale Promotoren, z.B. RSV-Promotor, CMV Immediate Early Promotor/Enhancer, SV40-Promotor, oder gewebespezifische, dabei vor allem leberspezifische Promotoren, z.B. der humane Albumin-Promotor (Ponder et al., Hum. Gene Ther. 2 (1991), 41-52), der humane α-1-Antitrypsin Promotor/Enhancer (Shen et al., DNA 8 (1989), 101-108), der PEPCK-Promotor (Ponder et al., Hum. Gene Ther. 2 (1991), 41-52) oder HBV-abgeleitete Hybrid-Promotoren, z.B. EIImCMV-Promotor (Löser et al., Biol. Chem. Hoppe-Seyler 377 (1996), 187-193). Weiterhin umfassen die Expressionsregulationssequenzen günstigerweise ein Polyadenylierungssignal, beispielweise das des Rinder-Wachstumshormongens (Goodwin & Rottman, J. Biol. Chem. 267 (1992); 16330-16334), das der frühen Transkriptionseinheit von SV40 (van den Hoff et al., Nucleic Acids Res. 21 (1993), 4987-4988) oder das des Herpes Simplex Thymidin-Kinase-Gens (Schmidt et al., Mol. Cell. Biol. 10, (1990), 4406-4411.

Der virale Gentranfervektor kann zum Transfer heterologer Nukleinsäuren in permissive Zellen, Zellverbände, Organe und Organismen, insbesondere zur Gentherapie oder zur Vakzinierung eingesetzt werden. Zum Ziel einer Gentherapie kann die genomische Sequenz oder die cDNA eines Gens verwendet werden, dessen Produkt in dem zu behandelnden Patienten fehlt, in unphysiologischen Mengen auftritt und defekt ist. Man kann auch einen Teil einer genomischen Sequenz einsetzen, die eine Mutation im Zielgen überspannt und mit dieser homolog rekombiniert werden kann. Zum Ziel einer Tumorgentherapie können verschiedene Gene, die ein verlangsamtes Wachstum oder ein Abtöten der Tumorzellen gegebenenfalls in Kombination mit Pharmaka oder durch Immunstimulation bewirken, eingesetzt werden. Zum Ziel einer Vakzinierung können ein oder mehrere gegebenenfalls veränderte Gene eines pathogenen Organismus, gegen den eine Immunisierung erreicht werden soll, eingesetzt werden.

Bevorzugte konkrete Beispiele für Transgene sind zum Ziel einer Substitutions-Gentherapie Gene für sekretierte Serumfaktoren (z.B. humane Blutgerinnungsfaktoren IX (FIX) und VIII (FVIII), Erythropoietin (Epo) α-1-Antitrypsin (AAT)), sowie Gene für Proteine, die bei Muskelerkrankungen einsetzbar sein könnten (z.B. Dystrophin, Utrophin), und das bei Morbus Wilson defekte Gen (ATP7B). Zum Ziel einer Tumortherapie sind bevorzugte konkrete Transgene Tumorsuppressorgene wie p16 oder p53 (einzeln oder in Kombination, z.B. P16/p53), Gene für verschiedene Interleukine (einzeln oder in Kombination, z.B. IL2/IL7) sowie Suizid-Gene, z.B. Herpes Simplex Virus Typ I Thymidin-Kinase (HSV-TK).

Die erfindungsgemäßen Gentransfervektoren sind nichthumane Adenovirusvektoren mit einem zumindest partiell deletierten viralen Genom. Das Adenovirus kann - wie bereits zuvor ausgeführt - aus beliebigen nichthumanen Spezies stammen. Bevorzugt stammt das Adenovirus vom Schaf oder Rind, wobei das Schafadenovirus OAV-Isolat 287 besonders bevorzugt ist.

In einer bevorzugten Ausführungsform enthält das genetische Material des Vektors (a) als virale Sequenzen zwei Inverted Terminal Repeats und eine oder mehrere Verpackungssequenzen und (b) bis zu ca. 30 kBp Fremd DNA. Weiterhin kann das genetische Material eine oder mehrere Matrix-Attachment-Regionen (MAR) enthalten. Es können verschiedene Matrix-Attachment-Regionen eingesetzt werden, beispielsweise die in einem Intron des Gens der humanen Hypoxanthin-Guanin-Phosphoribosyltransferase (HPRT) gelegene (Sykes et al., Mol. Gen. Genet. 212 (1988), 301-309) oder die im menschlichen Interferon-ß-Gen (Piechazek et al., Nucleic Acids Res. 27 (1999), 426-428) enthaltende verwendet werden. Neben dem kodierenden Transgen enthält das genetische Material des Transfervektors beispielsweise als Rückgrat nichtproteinkodierende Nukleinsäuren, die vorzugsweise aus genomischer Säuger-DNA, insbesondere aus genomischer humaner DNA stammen können.

Noch ein weiterer Aspekt der Erfindung betrifft das genetische Material zur Verpackung in einem erfindungsgemäßen Vektor. Das genetische Material ist eine Nukleinsäure, vorzugsweise eine DNA. Das genetische Material kann zur Manipulation oder/und Amplifizierung in einen Klonierungsvektor wie zuvor beschrieben, insbesondere zweischen zwei Meganuklease-Erkennungsstellen in einen Cosmid-Vektor inseriert werden.

Noch ein weiterer Aspekt der Erfindung ist ein System zur Produktion der erfindungsgemäßen viralen Vektoren. Dieses System umfaßt
(a) das genetische Material wie zuvor definiert,
(b) ein Helfersystem zur Bereitstellung der zur Replikation und Verpackung des Vektors notwendigen Genprodukte sowie
(c) gegebenenfalls Mittel zur Gewinnung und Aufreinigung des Vektors.

Das Helfersystem umfaßt vorzugsweise eine Verpackungszellinie, d.h. eine Zellinie, die eine Replikation und Verpackung des erfindungsgemäßen viralen Vektors erlaubt. In einer bevorzugten Ausführungsform der Erfindung ist die Verpackungszellinie eine konfektionierte Verpackungszellinie, die konstitutiv oder induzierbar die nicht von viralen Vektor kodierten Genprodukte für die Replikation und Verpackung des Vektors bereitstellt. Die für diese Genprodukte kodierenden Nukleinsäuren können durch Transformation bzw. Transfektion auf geeigneten Vektoren, z.B. Plasmiden oder mittels homologer Rekombination in die Verpackungszellinie eingeführt werden. Die Verwendung einer konfektionierten Verpackungszellinie hat den Vorteil, daß keine Kontaminationen der viralen Vektoren durch Helferviren auftreten. Die Verpackungszellinie ist vorzugsweise eine Säugerzellinie von einer Spezies, die gleich wie die Spezies ist, von der der nichthumane virale Vektor stammt.

Alternativ kann das Helfersystem auch eine Verpackungszellinie in Kombination mit einem Helfervirus umfassen, wobei das Helfervirus die zur Replikation und Verpackung des Vektors notwendigen Genprodukte vollständig und teilweise bereitstellt. In dieser Ausführungsform wird die Verpackungszellinie so gewählt, daß sie die zur Replikation und Verpackung des Vektors notwendigen Genprodukte nicht oder nur teilweise bereitstellt.

Das Helfervirus ist - wie zuvor bereits ausgeführt - vorzugsweise ein Adenovirus aus einer nichthumanen Spezies, entweder ein Wildtypvirus oder ein modifiziertes Virus. Zweckmäßigerweise stammt das Helfervirus aus derselben oder einer verwandten Spezies wie der virale Vektor. So können als Helferviren beispielsweise Schafadenoviren, wie etwa ovine Mastadenoviren oder Atadenoviren, insbesondere das OAV-Isolat 287 oder davon abgeleitete Derivate verwendet werden. Alternativ können auch Rinderadenoviren, beispielsweise bovine Atadenoviren oder bovine Mastadenoviren eingesetzt werden.

Um die Reinheit des helferabhängigen nichthumanen Virusvektors zu erhöhen und mögliche Kontaminatinen durch Helferviren zu minimieren oder vollständig zu vermeiden, ist die Verwendung eines Helfersystems bevorzugt, in dem die Verpackung des Helfervirus gegenüber der des helferabhängigen nichthumanen Virus benachteiligt ist. Dies kann beispielsweise durch Verwendung eines partiell verpackungshinhibierten Helfervirus geschehen, insbesondere eines Helfervirus, dessen Verpackungssequenz inaktiv, z.B. zumindest partiell deletiert ist. Alternativ kann eine Verpackungszellinie, die eine ortsspezifische Rekombinase exprimiert, in Kombination mit einem Helfervirus verwendet werden, dessen Verpackungssignal von Erkennungsstellen für diese ortsspezifische Rekombinase flankiert wird, so daß bei Coinfektion der Verpackungszellinie mit dem Helfervirus und dem genetischen Material des viralen Vektors das Verpackungssignal des Helfervirus durch die ortsspezifische Rekombinase aus dem Virusgenom geschnitten wird, was zu einer Verpackungsdefizienz des Helfervirus führt. Wenn als Rekombinasegen das Gen für die Cre-Rekombinase verwendet wird, sind die Rekombinase-Erkennungsstellen loxP-Sequenzen.

Die Gewinnung und Aufreinigung des viralen Vektors aus der Helferzellinie kann auf bekannte Art und Weise (siehe z.B. Wold, W.M.S. (ed.): Adenovirus Methods and Protocols; Humana Press, Totowa, New Jersey, 1999) erfolgen. Vorzugsweise werden hierzu eine Cäsiumchlorid-Dichtegradientenzentrifugation oder/und eine affinitätschromatographische Trennung eingesetzt.

Der erfindungsgemäße Vektor kann zum Transfer von genetischem Material in eine Zielzelle und vorzugsweise zur Expression dieses genetischen Materials in der Zielzelle verwendet werden. Die Zielzelle ist bevorzugt eine humane Zelle. Es können jedoch auch nichthumane Zielzellen, insbesondere nichthumane Säugerzellen, beispielsweise für veterinärmedizinische Anwendungen oder Anwendungen in der Forschung, verwendet werden. Der Gentransfer kann in vitro, d.h. in kultivierten Zellen, aber auch in vivo, d.h. in lebenden Organismen oder spezifischen Geweben oder Organen derartiger Organismen erfolgen.

Der Vektor ist geeignet zur Herstellung eines Mittels für die Nukleinsäurevakzinierung oder eines Mittels für die Gentherapie oder insbesondere die Therapie von ererbten oder malignen Erkrankungen. Schließlich kann der Vektor auch zur Gewinnung von Proteinen durch Überexpression in kultivierten Zellen eingesetzt werden.

Durch die vorliegende Erfindung wird ein neuartiger Vektor für den Gentransfer bereitgestellt, der gegenüber bisher entwickelten viralen Vektoren wie Retroviren, humanen Adenoviren und Adeno-assoziierten Viren entscheidende Vorteile aufweist. Dazu gehören vor allem die Abwesenheit einer präexistierenden Immunantwort im Menschen gegen den nichthumanen Vektor, wodurch ein effektiver Gentransfer bei niedriger Vektordosis möglich wird. Weiterhin wird aufgrund der weitgehenden oder vollständigen Abwesenheit der viralen Genexpression in den Zielzellen das Risiko unerwünschter Nebenwirkungen minimiert. Aus dem gleichen Grund wird das Risiko einer immunologischen Eliminierung erfolgreich transduzierter Zielzellen verringert, was eine wichtige Voraussetzung für die langfristige Expression des Transgens ist. Der Vektor hat eine Kapazität für Fremd-DNA bis zu einer Größe von ca. 30 kBp, wodurch auch die Insertion großer genomischer Sequenzen, mehrerer Gene oder komplexer Regulationselementezurreguliertenoder/und gewebsspezifischen Genexpression möglich wird. Auf diese Weise kann das Risiko unerwünschter Nebenwirkungen durch aberrante Expressionsorte oder/und Expressionsstärken vermindert werden.

Die erfindungsgemäßen helferabhängigen nichthumanen Adenovirusvektoren ermöglichen somit, maßgeschneiderte Fremd- DNA, insbesondere therapeutische DNA mit einer Gesamtgröße bis zu etwa 30 kBp in Zielzellen zu transferieren bei gleichzeitiger Minimierung des Risikos unerwünschter Nebenwirkungen nach lokaler oder systemischer Applikation in vivo. Dadurch wird eine wesentliche Voraussetzung für eine erfolgreiche Prävention pathogen bedingter Erkrankungen bei Tier und Mensch sowie der Therapie genetischer und maligner Erkrankungen des Menschen geschaffen.

Die bevorzugte Verabreichungsform des Vektors hängt von der geplanten Anwendung ab. Für muskelgerichteten Gentransfer oder Transfer in einen soliden Tumor ist beispielsweise eine lokale Applikation des Vektors durch intramuskuläre/intratumorale Injektion vorzuziehen. Zum Gentransfer in andere Zielorgane oder -gewebe kann eine systemische Einbringung beispielsweise durch intraperitoneale, intraarterielle oder intravenöse Injektion erfolgen. Der gerichtete Transfer in spezielle Gewebe oder Organe kann im letzteren Fall entweder durch einen natürlichen bzw. modifizierten Tropismus des Vektors für betimmte Zelltypen oder durch Auswahl von Gefäßen, die das zu treffende Gewebe versorgen, erfolgen.

Die Dosierung kann erst nach genaueren Studien zur Effizienz des Gentransfers durch den jeweiligen Vektor entschieden werden.

Typischerweise werden 10⁷ bis 10¹³, z.B. 10⁹ bis 10¹¹ virale Partikel/Kg Körpergewicht eingesetzt. Die genaue Dosierung kann jedoch je nach der Art des Vektors, der Art und Schwere der Erkrankung und der Art der Verabreichung modifiziert werden.

Die Erfindung soll durch die nachfolgenden Abbildungen und Ausführungsbeispiele näher erläutert werden. Es zeigen:

| | |
|---|---|
| Abbildung 1 | eine schematische Darstellung der Klonierung des Genoms des nichthumanen Adenovirus OAV 287 in den Cosmidvektor pMVKpn, |
| Abbildung 2 | eine schematische Darstellung der Experimente zur Lokalisierung der Verpackungssequenz von OAV 287, |
| Abbildung 3 | schematische Darstellung des Basisvektors pMOAV2.8vk zur Insertion von Transgenen und anschließenden Freisetzung eines linearen Genoms eines helferabhängigen Virus-Vektors durch Meganukleaseverdau. |

### Beispiele

### 1. Klonierung des Genoms eines nichthumanen Virus in einen Cosmidvektor und Charakterisierung der Lage des Verpackungssignals

Es wurde der Cosmid-Vektor pMVKpn aus pMVX-lacZ konstruiert. Dieser Vektor enthält unter anderem einen von Schnittstellen für Meganuklease I-Scel umrahmten bakteriellen Teil, bestehend aus einem bakteriellen Replikationsursprung (ColE1) und einem bakteriellen Ampicillin-Resistenzgen (beide aus pBluescript, Stratagene), sowie dem cos-Element aus Resistenzgen (beide aus pBluescript, Stratagene), sowie dem cos-Element aus dem Cosmid-Vektor pWE15 (Stratagene). Zur Konstruktion von pMVKpn wurde dieser bakterielle Teil durch I-Scel-Verdau aus pMVX-lacZ freigesetzt. Zur Konstruktion von pMVKpn wurde zwischen die I-Scel-Enden ein Linker inseriert, der durch Hybridisierung des synthetischen Oligonukleotids SceKpn (5'-CCCTAGGTACCTAGGGATAACAG-3') gewonnen worden war. Der Linker bewirkte dabei die Wiederherstellung der beiden I-Scel-Erkennungssequenzen sowie die Insertion einer Kpnl-Erkennungsstelle zwischen den beiden wiederhergestellten I-Scel-Erkennungssequenzen.

Der Vektor pMVKpn enthält als wichtige funktionelle Elemente das Verpackungssignal (cos-Signal) des Phagen Lambda sowie zwei direkt benachbarte Schnittstellen für die Meganuklease I-Scel mit einer dazwischen liegenden unikalen Schnittstelle für Kpnl. In letztere können unter Verwendung hocheffizienter Cosmid-Klonierungsmethoden - Verpackung der Ligationsprodukte in Lambda-Phagen-Köpfe und Infektion von E.coli - vollständige virale Genome unterschiedlicher Provenienz (z.B. aus infektiösen Viren) in der Größenordnung von 25 bis 40 KB inseriert werden. Die so kloniertenVirusgenome können charakterisiert oder manipuliert werden. Über die Meganuklease-Schnittstellen können infektiöse, lineare Genome mit freien ITRs aus den resultierenden Konstrukten freigesetzt werden. Durch die Länge der Erkennungssequenz (18 Bp) ist das Auftreten einer I-Scel-Schnittstelle im viralen Genom praktisch auszuschließen.

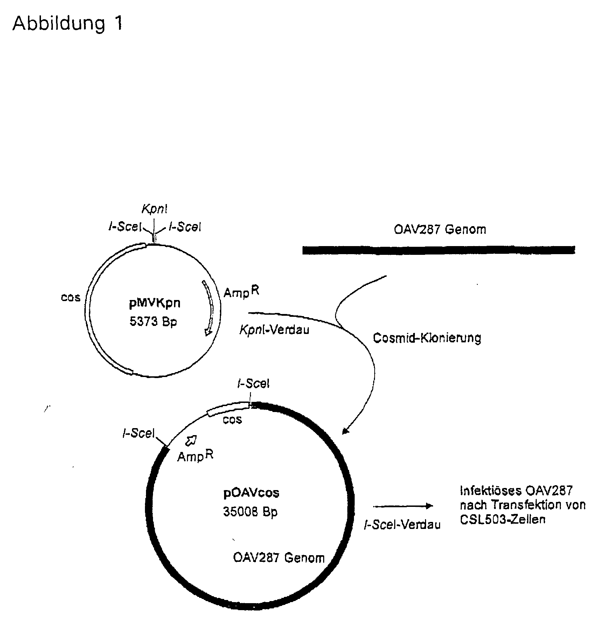

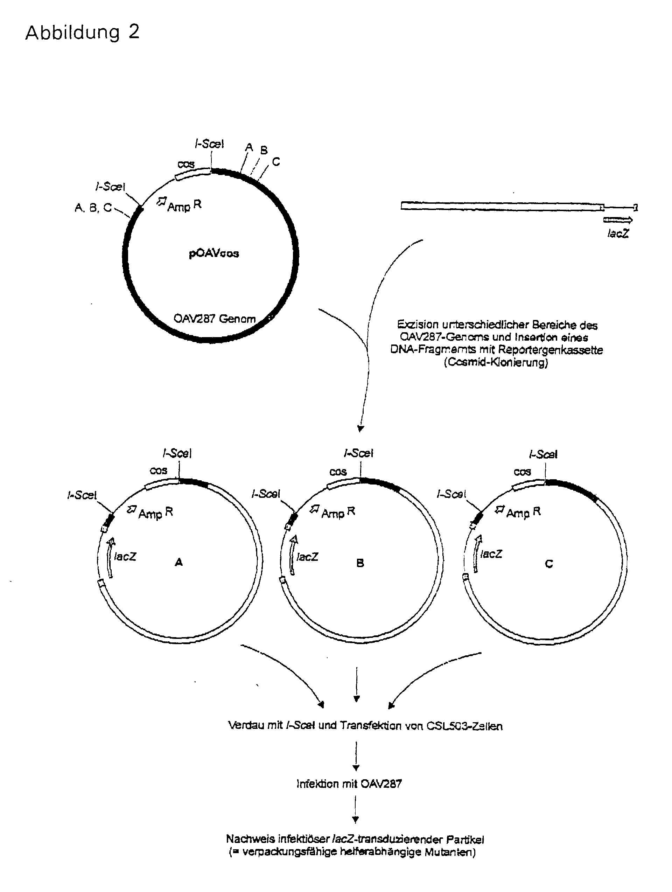

Das Genom des Schaf-Adenovirus OAV287 wurde in die Kpnl-Schnittstelle pMVKpn über eine Cosmid-Klonierung inseriert, wodurch das Konstrukt pOAVcos entstand. Nach Transfektion von Meganuklease I-Scel-verdautem pOAVcos auf permissive CSL503 Zellen wurde effizient infektiöses OAV287 erzeugt (Abbildung 1). Dieses Konstrukt ermöglicht die Charakterisierung und Manipulation des OAV 287 Genoms (z.B. Deletionen verschiedener Regionen und/oder Insertion von Transgenen) aber auch die Lokalisierung des bislang nicht charakterisierten Verpackungssignals.

Zur Charakterisierung der Lage des Verpackungssignals von OAV287 wurden unter Verwendung der Cosmidklonierungstechnik unterschiedliche Bereiche des viralen Genoms ausgeschnitten und durch Spacer-DNA und eine konstitutive lacZ-Expressionskassette ersetzt.

Die Konstrukte wurden in CSL503 Zellen transfiziert, die Zellen anschließend mit OVA287 als Helfervirus infiziert und nach Auftreten des OAV287-vermittelten cytopathischen Effekts lysiert. Transduktion des lacZ-Gens nach Infektion von Zellen mit dem Lysat zeigte an, daß in dem jeweils verwendeten Konstrukt das Verpackungssignal nicht deletiert worden war.

Auf diese Weise konnte die Lage des Verpackungssignals auf die 5'-terminalen 2,8 KBp oder die 3'-terminalen 1 KBp des Virusgenoms eingegrenzt werden.

### 2. Herstellung von Basisvektoren zur vereinfachten Insertion von Transgenen in die helferabhängigen nichthumanen Virusvektoren

Nach der partiellen Charakterisierung der Lage des Verpackungssignals von OAV287 (Ausführungsbeispiel 1), wurde ein Klonierungsvektor zur vereinfachten Insertion von Transgenen in einen helferabhängigen Schaf-Adenovirus-Vektor konstruiert (pMOAV2.8vk). Dazu wurde zwischen die 5'-terminalen 2,8 kBp und die 3'-terminalen 1 kBp eines nichthumanen Adenovirus, 17,3 kBp nichtkodierende genomische DNA aus dem menschlichen X-Chromosom sowie eine lacZ-Expressionskassette zur Detektion und Titration der abzuleitenden helferabhängigen Viren inseriert.

Das X-chromosomale Fragment hat zwei Funktionen: Zum einen bringt es die Größe des Genoms des helferabhängigen Schaf-Virusvektors auf die zur Verpackung in virale Capside notwendige Mindestgröße und zum anderen stellt es geeignete Schnittstellen zur Insertion von Transgenen zur Verfügung, mit oder ohne gleichzeitige Excision von Teilen des Fragments (Insertionsstelle mit Größenausgleich). Der Basisvektor hat des weiteren eine cos-Erkennungssequenz, die zur Insertion von Transgenen die Cosmidklonierungs-Technik einsetzbar macht. Desweiteren sind die 3'-und 5'-Enden des Schaf-Adenovirusvektors im Basisvektor durch Schnittstellen für die Meganuklease I-Scel flankiert, was die Freisetzung linearer Genome mit terminalen ITRs ermöglicht.

Zur Insertion des OAV287-Genoms in pMVKpn wurde das Genom von OAV287 (Genbank Acc No. U40839) als Kpnl-Fragment aus pOAVpoly (enthält Wildtyp-OAV287-Genom mit einem Polylinker) freigesetzt und über eine Cosmid-Klonierung in den unikalen Kpnl-Ort von pMVKpn inseriert, resultierend in pOAVcos.

Anschließend wurde ein Großteil des OAV-Genoms durch ein X-chromosomales Stuffer-Fragment sowie eine E.coli lacZ-Expressionskassette ersetzt. Über Doppelverdau mit Bst10071 und Sall wurde aus pOAVcos das OAV-Genom zwischen Bp 3956 und 28729 herausgeschnitten und durch ein - aus dem Vektor pMVX-lacZ über Sal/I/Nrul-Spaltung freigesetztes - 26294 Bp-Fragment mit 22069 Bp nichtkodierender humaner genomischer X-chromosomaler DNA (entsprechend Bp 18475-40511 von Genbank Accession No. U82672) und einer 5'-terminal gelegenen 4225-Bp-Expressionskassette des E.coli lacZ Gens unter Kontrolle des RSV-Promotors und des SV40-Polyadenylierungssignals ersetzt (Cosmid-Klonierung). Dies resultierte in Vektor pMOAV4.0, in dem die beiden Erkennungssequenzen für die Meganuklease I-Scel ein insgesamt ca. 32 kBp großes Fragment umrahmen, in dem 3956 Bp vom 5'-Ende und 896 Bp vom 3'-Ende von OAV287 ein humanes X-chromosomales Fragment sowie eine lac-Z-Expressionskassette umrahmen.

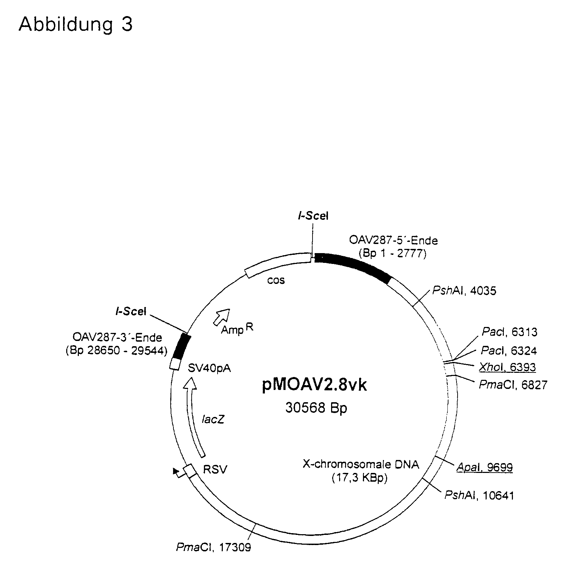

Ausgehend von pMOAV4.0 wurde durch Exzision eines 1206 Bp-Pmel-Fragments (Positionen 2739 und 3945 im OAV-Genom) der 5'-Bereich von OAV auf 2739 Bp verkürzt, resultierend in pMOAV2.8. Durch Exzision eines 4757 Bp großen Xhol-Fragments im X-chromosomalen Bereich von pMOAV2.8 wurde schließlich pMOAV2.8vk gewonnen (vgl. Abbildung 3).

### 3. Herstellung eines helferabhängigen nichthumanen Virusvektors mit einem Transgen

Ausgehend von den Basisvektoren zur Generierung helferabhängiger Schaf-Adenovirusvektoren (Ausführungsbeispiel 2) erfolgt die Insertion von Transgen-Expressionskassetten je nach deren Anzahl und Größe in eine oder mehrere geeignete Schnittstellen der Klonierungsstelle mit oder ohne Größenausgleich über die Cosmidklonierungstechnik. So wurde eine Expressionskassette für das humane Alpha-I-Antitrypsin als Xhol-Fragment aus dem bei Hofmann et al., J. Virol. 73 (1999), 6930-6936) beschriebenen Plasmid pRSVhAAT freigesetzt und in die Xhol-Stelle von pMOAV2.8vk inseriert. Die Gesamtgröße des rekombinanten Virusgenoms blieb dabei unter 27 kBp. Das so generierte rekombinante Virusgenom wurde aus dem Konstrukt durch Verdau mit Meganuklease I-Scel freigesetzt und in das Helfersystem transfiziert. Das Helfersystem besteht aus CSL503-Zellen als Verpackungszellinie und OAV287 (Genomgröße ~ 30 kBp) als Helfervirus. Aus dem resultierenden Gemisch wurde der helferabhängige Schaf-Adenovirus-Vektor vom Helfervirus durch CsCI-Dichtegradienten-Zentrifugation - basierend auf unterschiedlicher Dichte aufgrund unterschiedlicher Genomgrößen - gereinigt.

## Patentansprüche

1. Klonierungsvektor umfassend
(a) eine Verpackungssequenz eines Bakteriophagen,
(b) mindestens eine Klonierungsstelle zur Insertion heterologer Nukleinsäuresequenzen,
(c) mindestens zwei Schnittstellen für eine Restriktionsendonuklease, die beidseitig die Klonierungsstelle (b) umrahmen,
(d) einen bakteriellen Replikationsursprung, und
(e) ein bakterielles Selektionsmarkergen.

2. Vektor nach Anspruch 1,
**dadurch gekennzeichnet**,
daß die Bakteriophagen-Verpackungssequenz von einem lambdoiden Phagen, insbesondere vom Phagen Lambda (cos-Sequenz) stammt.

3. Vektor nach einem der Ansprüche 1 oder 2,
**dadurch gekennzeichnet**,
daß Schnittstellen (c) für die Meganuklease I-Scel vorliegen.

4. Vektor nach einem der Ansprüche 1 bis 3,
**dadurch gekennzeichnet**,
daß das Selektionsmarkergen (e) ein Antibiotikaresistenzgen umfaßt.

5. Vektor nach einem der Ansprüche 1 bis 4,
**dadurch gekennzeichnet**,
daß er inseriert in der Klonierungsstelle (b) das Genom eines nichthumanen Adenovirus enthält.

6. Verwendung eines Klonierungsvektors nach einem der Ansprüche 1 bis 5 zur Herstellung von partiell deletierten Genomen nichthumaner Adenoviren.

7. Verwendung nach Anspruch 6 zur Identifizierung und Charakterisierung der Verpackungssequenzen nichthumaner Adenoviren.

8. Verwendung nach Anspruch 6 oder 7,
**dadurch gekennzeichnet**,
daß
(a) ein vorbestimmter Bereich des in den Klonierungsvektor inserierten viralen Genoms deletiert und durch eine heterologe Nukleinsäure umfassend eine Reportergenkassette ersetzt wird,
(b) die gemäß (a) erzeugten Konstrukte in ein Helfersystem eingebracht werden, das zur Replikation und Verpackung des nichthumanen Adenovirus notwendigen Genprodukte bereitstellt,
(c) bestimmt wird, ob mit dem System gemäß (b) nichthumane Adenoviruspartikel entstehen, die das Reportergen enthalten, und
(d) gegebenenfalls die Schritte (a), (b) und (d) wiederholt werden, bis die neben den Inverted Terminal Repeats (ITRs) für eine Verpackung notwendigen cis-Sequenzen des Virusgenoms (Verpackungssequenzen) charakterisiert sind.

9. Verwendung nach Anspruch 6 zur Herstellung eines Basisvektors.

10. Basisvektor, insbesondere zur Herstellung eines viralen Vektors für den Gentransfer, umfassend
(a) eine Verpackungssequenz eines Bakteriophagen,
(b) virale Sequenzen umfassend zwei Inverted Terminal Repeats (ITRs) und eine oder mehrere Verpackungssequenzen eines nichthumanen Adenovirus, wobei die viralen Sequenzen nicht in der Lage sind, eine helferunabhängige virale Replikation und Verpackung in einer permissiven Zellinie zu bewirken,
(c) eine Klonierungsstelle zur Insertion von heterologer DNA und gegebenenfalls eine Reportergenkassette, die innerhalb der viralen Sequenzen (b) liegen,
(d) mindestens zwei Schnittstellen für eine Restriktionsendonuklease, die beidseitig die viralen Sequenzen (a) umrahmen,
(e) einen bakteriellen Replikationsursprung und
(f) ein bakterielles Selektionsmarkergen,
wobei die Sequenzen (a), (e) und (f) außerhalb des von den Schnittstellen (d) umrahmten Sequenzbereichts liegen.

11. Vektor nach Anspruch 10,
**dadurch gekennzeichnet**,
daß er eine Reportergenkassette innerhalb der Schnittstellen (d) enthält.

12. Vektor nach Anspruch 10 oder 11,
**dadurch gekennzeichnet**,
daß die Klonierungsstelle (c) eine heterologe DNA zum Größenausgleich umfaßt.

13. Vektor nach Anspruch 9 bis 12,
**dadurch gekennzeichnet**,
daß die heterologe DNA eine nichtkodierende genomische Säuger-DNA umfaßt.

14. Vektor nach Anspruch 11,
**dadurch gekennzeichnet**,
daß die Reportergenkassette das E.coli lacZ-Gen in exprimierbarer Form umfaßt.

15. Vektor nach einem der Ansprüche 10 bis 14,
**dadurch gekennzeichnet**,
daß er in der Klonierungsstelle (c) ein Transgen inseriert enthält.

16. Verwendung des Vektors nach einem der Ansprüche 10 bis 15 zur Herstellung eines helferabhängigen nichthumanen adenoviralen Gentransfervektors.

17. Viraler Gentransfervektor umfassend die Hülle eines nichthumanen Adenovirus und darin verpacktes genetisches Material, das
(a) virale Sequenzen umfassend zwei Inverted Terminal Repeats (ITRs) und eine oder mehrere Verpackungssequenzen eines nichthumanen Adenovirus,
(b) eine oder mehrere Nukleinsäuresequenzen, die für bezüglich des nichthumanen Adenovirus heterologe Peptide oder Polypeptide kodieren, operativer Verknüpfung mit Expressionskontrollsequenzen und
(c) gegebenenfalls nichtkodierende DNA zum Größenausgleich enthält,
wobei das genetische Material nicht in der Lage ist, eine helferunabhängige virale Replikation und Verpackung in einer permissiven Zellinie zu bewirken.

18. Vektor nach Anspruch 17,
**dadurch gekennzeichnet**,
daß das genetische Material mit Ausnahme von den für Replikation und Verpackung in die Hülle notwendigen cis-Elementen frei von funktionellen Nukleinsäuresequenzen des nichthumanen Adenovirus ist.

19. Vektor nach Anspruch 17 oder 18,
**dadurch gekennzeichnet**,
daß das Virus ein Adenovirus aus einer nicht-humanen Spezies ausgewählt aus Säugern und Vögeln ist.

20. Vektor nach Anspruch 19,
**dadurch gekennzeichnet**,
daß das Virus ein Adenovirus vom Schaf oder Rind ist.

21. Vektor nach Anspruch 20,
**dadurch gekennzeichnet,**
daß das Adenovirus vom Schaf ein ovines Mastadenovirus oder ein ovines Atadenovirus ist.

22. Vektor nach Anspruch 20 oder 21,
**dadurch gekennzeichnet**,
daß das Adenovirus vom Schaf das OAV-lsolat 287 ist.

23. Vektor nach Anspruch 20,
**dadurch gekennzeichnet**,
daß das Adenovirus vom Rind ein bovines Mastadenovirus oder ein bovines Atadenovirus ist.

24. Vektor nach einem der Ansprüche 16 bis 23,
**dadurch gekennzeichnet**,
daß das genetische Material
(a) als virale Sequenzen zwei Inverted Terminal Repeats (ITRs) und eine oder mehrere Verpackungssequenzen und
(b) bis zu ca. 30 kBp Fremd-DNA enthält.

25. Vektor nach einem der Ansprüche 16 bis 24,
**dadurch gekennzeichnet**,
daß das genetische Material eine oder mehrere Matrix-Attachment-Regionen (MAR) enthält.

26. Vektor nach einem der Ansprüche 16 bis 25,
**dadurch gekennzeichnet**,
daß das genetische Material als Rückgrat nichtproteinkodierende Nukleinsäuren enthält.

27. Genetisches Material zur Verpackung in einem Vektor nach einem der Ansprüche 16 bis 26.

28. Genetisches Material nach Anspruch 27 inseriert in einem Klonierungsvektor nach einem der Ansprüche 1 bis 5 oder in einem Basisvektor nach einem der Ansprüche 10 bis 15.

29. System zur Produktion der viralen Vektoren nach einem der Ansprüche 16 bis 26, umfassend
(a) das genetische Material nach Anspruch 27 oder 28,
(b) ein Helfersystem zur Bereitstellung der zur Replikation und zur Verpackung des Vektors notwendigen Genprodukte sowie
(c) gegebenenfalls Mittel zur Gewinnung und Aufreinigung des Vektors.

30. System nach Anspruch 29,
**dadurch gekennzeichnet**,
daß das Helfersystem eine konfektionierte Verpackungszellinie umfaßt.

31. System nach Anspruch 30,
**dadurch gekennzeichnet**,
daß die Verpackungszellinie konstitutiv oder induzierbar die nicht vom Vektor selbst kodierten Genprodukte für die Replikation und Verpackung des Vektors bereitstellt.

32. System nach einem der Ansprüche 29 bis 31,
**dadurch gekennzeichnet**,
daß das Helfersystem eine Verpackungszellinie und ein Helfervirus umfaßt.

33. System nach Anspruch 32,
**dadurch gekennzeichnet**,
daß das Helfervirus die zur Replikation und Verpackung des Vektors notwendigen Genprodukte vollständig oder teilweise bereitstellt.

34. System nach Anspruch 32 oder 33,
**dadurch gekennzeichnet**,
daß die Verpackungszellinie die zur Replikation und Verpackung des Vektors notwendigen viralen Genprodukte nicht oder nur teilweise bereitstellt.

35. System nach Anspruch 32 oder 33,
**dadurch gekennzeichnet**,
daß das Helfervirus ein Adenovirus aus einer nicht-humanen Spezies ist.

36. System nach Anspruch 35,
**dadurch gekennzeichnet**,
daß das Helfervirus ein Adenovirus vom Schaf ist.

37. System nach Anspruch 36,
**dadurch gekennzeichnet**,
daß das Helfervirus vom Schaf ein ovines Mastadenovirus oder ein ovines Atadenovirus ist.

38. System nach Anspruch 36 oder 37,
**dadurch gekennzeichnet**,
daß das Helfervirus vom Schaf das OAV-lsolat 287 ist.

39. System nach Anspruch 35,
**dadurch gekennzeichnet**,
daß das Helfervirus ein Adenovirus vom Rind ist.

40. System nach Anspruch 39,
**dadurch gekennzeichnet**,
daß das Helfervirus vom Rind ein bovines Mastadenovirus oder ein bovines Atadenovirus ist.

41. System nach einem der Ansprüche 32 bis 40,
**dadurch gekennzeichnet**,
daß das Helfervirus partiell verpackungsinhibiert ist.

42. System nach Anspruch 41,
**dadurch gekennzeichnet**,
daß die Verpackungssequenz des Helfervirus partiell deletiert ist.

43. System nach einem der Ansprüche 32 bis 42,
**dadurch gekennzeichnet**,
daß die Verpackungssequenz des Helfervirus von Erkennungsstellen für eine ortsspezifische Rekombinase umrahmt wird.

44. System nach Anspruch 39,
**dadurch gekennzeichnet**,
daß die Verpackungszellinie ein Gen für eine ortsspezifische Rekombinase exprimiert.

45. System nach Anspruch 43 oder 44,
**dadurch gekennzeichnet**,
daß die Erkennungsstellen für eine Rekombinase loxP-Sequenzen sind und das Rekombinasegen das Gen für Cre-Rekombinase ist.

46. System nach einem der Ansprüche 29 bis 45,
**dadurch gekennzeichnet**,
daß die Mittel zur Gewinnung und Aufreinigung des Vektors eine Cäsiumchlorid-Dichtegradientenzentrifugation oder/und affinitätschromatographische Trennung umfassen.

47. Verwendung des Vektors nach einem der Ansprüche 16 bis 26 zum Transfer von genetischem Material in eine Zielzelle.

48. Verwendung nach Anspruch 47 weiterhin umfassend die Expression des genetischen Materials in der Zielzelle.

49. Verwendung nach Anspruch 47 oder 48,
**dadurch gekennzeichnet,**
daß die Zielzelle eine humane Zelle ist.

50. Verwendung nach einem der Ansprüche 47 bis 49 zur NukleinsäureVakzinierung.

51. Verwendung nach einem der Ansprüche 47 bis 49 zur Gentherapie.

52. Verwendung nach Anspruch 51 zur Therapie von ererbten oder malignen Erkrankungen.

53. Verwendung nach einem der Ansprüche 47 bis 49 zur Gewinnung von Proteinen durch Überexpression in der Zielzelle.
